(19) Europäisches Patentamt / European Patent Office / Office européen des brevets

(11) **EP 1 900 809 A1**

(12) **EUROPEAN PATENT APPLICATION**
published in accordance with Art. 153(4) EPC

(43) Date of publication:
**19.03.2008 Bulletin 2008/12**

(51) Int Cl.:
**C12N 5/00** (2006.01)   **A61L 27/00** (2006.01)
**C12N 5/06** (2006.01)

(21) Application number: **06746345.5**

(22) Date of filing: **12.05.2006**

(86) International application number:
**PCT/JP2006/309548**

(87) International publication number:
**WO 2006/123579 (23.11.2006 Gazette 2006/47)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.05.2005 JP 2005143443**

(71) Applicants:
• **National University Corporation Nagoya University**
  **Nagoya-shi, Aichi 464-8601 (JP)**
• **Arblast Co., Ltd.**
  **Kobe city, Hyogo 650-0047 (JP)**

(72) Inventors:
• **UEDA, Minoru,**
  **Nat. Univ. Corp. Nagoya University**
  **Nagoya-shi Aichi 4648601 (JP)**
• **YAMADA, Yoichi,**
  **Nat. Univ. Corp. Nagoya University**
  **Nagoya-shi, Aichi 4648601 (JP)**
• **SHIMA, Nobuyuki**
  **4F, New Rokkou Heights**
  **Kobe-shi, Hyogo 6570015 (JP)**

(74) Representative: **Müller Fottner Steinecke**
  **Rechtsanwälte Patentanwälte,**
  **Postfach 31 01 40**
  **80102 München (DE)**

(54) **METHOD OF PREPARING CELL FOR BONE TISSUE FORMATION AND APPLICATION OF CELL FOR BONE TISSUE FORMATION**

(57)   It is intended to provide a method of efficiently and stably preparing a cell having a bone tissue formation ability by a simple operation. Further, it is intended to provide a method of preparing a composition for bone tissue formation with high safety and an excellent therapeutic effect by a simple operation. The cell for bone tissue formation is obtained by (1) culturing bone marrow after isolating it from a living body, diluting it at a predetermined dilution ratio and inoculating it to a culture vessel, (2) culturing the remaining adhesive cells after removing floating components, (3) inducing differentiation of proliferated cells to bone cells and (4) recovering the cells. The thus obtained cells, a thrombin solution, platelet-rich plasma and air are mixed at a predetermined mixing ratio (based on volume) in the presence of calcium ions and the mixture is gelled, whereby a gelled composition is obtained.

Fig.1

| | |
|---|---|
| Collect bone marrow fluid | Step 1 |
| Recover bone marrow fluid in centrifugation tube | Step 2 |
| Plate and culture bone marrow fluid | Step 3 |
| Replace medium with new one (remove blood cells) | Step 4 |
| Passage culture and cell proliferation | Step 5 |
| Induce differentiation | Step 6 |
| Recover cells (centrifugation) | Step 7 |

**Description**

TECHNICAL FIELD

The present invention relates to a method for preparing cells that can be used for repairing or regenerating (reconstructing) bone tissue, a method for preparing a composition for forming bone tissue by using the cells, and a technique using the composition for forming bone tissue.

BACKGROUND ART

In the fields of craniofacial surgery, plastic surgery and orthopedic surgery, many researches have been carried out for the purpose of reconstruction and repair of bone defects. However, in use and preparation of biocompatible materials for both soft tissue and hard tissue, there are many problems left to be clinically resolved. To date, excised autologous tissue including a bone fragment, fat, and fascia has been frequently used. However, treatment methods to date have had individual problems. For example, the supply amount of appropriate autologous materials is limited, and autologous materials are not sufficient for the intended reconstruction operation because of contraction of a donor site and sometimes because the tissue quality is low or it is difficult to shape materials into a graft. The supply amount of allogenic materials is also limited due to the shortage of donors. On the other hand, use of artificial repairing products increases the sensitivity to infection. It also has problems, for example, they invade into the peripheral portions, and a long-time interaction with the physiological function of a host is not clear.
As a means for resolving such problems in organ transplantation, Vacanti et al. (non-patent documents 1 to 3) proposed a new technology called "tissue engineering" in which a construct formed by combining a biocompatible scaffold and growth factors with isolated cells is used so as to achieve morphogenesis of a neoblastic tissue. In general, this technique requires invasive transplantation of a cell-scaffold construct. If a system, in which this technique is applied to osteoblast and a cell-scaffold construct can be transplanted with low invasiveness, is developed, the application range of the tissue engineering is broadened to many fields of, for example, craniofacial reconstruction, plastic surgery, orthopedic surgery, and the like.
It is thought that the mesenchymal stem cells (MSCs) can be replicated as undifferentiated cells and are multipotent cells having a differentiation potency to the mesenchymal tissue line including bone, cartilage, fat, tendon, muscle, and bone marrow stromal tissue (non-patent documents 4 and 5). Therefore, the mesenchymal stem cells receive wide attention because of its high potential usefulness in the field of tissue engineering.
To date, it has been shown that by using MSCs singly, the bone tissue could be repaired (non-patent documents 5 and 6). However, if a defect portion is filled with only MSCs, very large amount of MSCs are needed. Furthermore, it is difficult to retain MSCs in a bone defect portion. For these reasons, recently, many researchers have attempted to use artificial materials such as ceramics as a scaffold (non-patent document 7).
In the preceding research, the present inventors have investigated a bone forming ability as a scaffold of new bioabsorbable β-triphosphate (β-TCP) combined with MSCs and have compared it with that of hydroxyapatite (HA) (non-patent document 8). The result of this research has revealed that this scaffold has a bone forming ability but it is fragile. It has also revealed that newly formed bone is prevented from entering into the bone defect portion due to the shortage of through holes, low absorbing property of ceramics and absence of the bone induction property. Next, the present inventors have attempted a technique using a low invasive jelly-like flexible material that can be used for delivering autologous bone in order to treat or reconstruct a bone defect caused by osteoporosis, periodontitis or excise of tumor. Firstly, as an injectable scaffold, fibrin glue was used. With an injectable MSCs / β-TCP- fibrin glue mixture, it was possible to form a three-dimensional scaffold necessary for excellent transplantation and survival of osteoblast (non-patent document 9). An example in which a bone inducing material, a coagulating agent of a graft, and fibrin as a graft material were used, has been reported (non-patent documents 10 and 11). Furthermore, the importance of the growth factors found in the autologous fibrin glue is described (non-patent documents 12 to 14). Then, the present inventors have thought of using a platelet-rich plasma (PRP) gel made of a mixture including a growth factor and a modified autologous fibrin glue. The PRP gel can be obtained by mixing, platelet-rich plasma, which are obtained by centrifuging the autologous whole blood under a plurality of different conditions, thrombin and calcium chloride. The crucial difference between the PRP gel and the fibrin glue is in that the PRP gel includes platelets at high concentration and has an original fibrinogen concentration. Once the platelet is activated in the presence of thrombin, it functions of releasing a large number of factors and forming a fibrin mass, thereby forming a scaffold. The use of the PRP has some advantages, for example, the bone regeneration is enhanced and promoted, and more quick and predictable curing of soft tissue can be achieved. By using monoclonal antibody techniques, transforming growth factor β1 (TGF-β1), transforming growth factor β2 (TGF-β2) and platelet-derived growth factor (PDGF) receptors are found in the medullary bone (non-patent documents 12 to 14). With the study using monoclonal antibodies, it is shown that PDGF and TGF-β are present at high concentration in the plasma. Thus, these factors have been proven to be present in the original plasma used for obtaining the autologous fibrin.

Note here that technologies relating to the present application are disclosed in International Publication WO 02/17983 pamphlet and International Publication WO 02/40071 pamphlet (patent documents 1 and 2).

[Patent document 1] International Publication WO 02/17983 pamphlet

[Patent document 2] International Publication WO 02/40071 pamphlet

[Non-patent document 1] Langer, R., and Vacanti, J.P. Tissue engineering. Science 260, 920, 1993.

[Non-patent document 2] Vacanti, J.P., Morse, M.A., Saltzman, W.M., Domb, A.J., Perez-Atayde, A., and Langer, R. Selective cell transplantation using bioabsorbable artificial polymers as matrices. J Pediatr Surg 23, 3, 1988.

[Non-patent document 3] Vacanti, J.P. Beyond transplantation. Arch. Surg 123, 545, 1998.

[Non-patent document 4] Pittenger, M.F., Mackay, A.M., Beck, C.B., Jaiswal, R.K., Douglas, R., Mosca, J.D., Moorman, M.A., Simonetti, D.W., Craig, S., and Marshak, D.R. Multilineage potential of adult human mesenchymal stem cells. Science 284, 143, 1999 .

[Non-patent document 5] Owen, M., and Friedenstein, A.J. Stromal stem cells: Marrow derived osteogenic precursors. Ciba Found Symp 136, 42, 1998.

[Non-patent document 6] Kadiyala, S., Young, R.G., Thiede, M.A., and Bruder, S.P. Culture expanded canine mesenchymal stem cells possess osteochondrogenic potential in vivo and in vitro. Cell Transplant 6, 125, 1997.

[Non-patent document 7] Bruder, S.P., Kurth, A.A., Shea, M., Hayes, W.C., Jaiswal, N., and Kadiyala, S. Bone regeneration by implantation of purified, culture-expanded human mesenchymal stem cells. J Orthop Res 16, 155, 1998.

[Non-patent document 8] Boo, J.S., Yamada, Y., Hibino, Y., Okazaki, Y., Okada, K., Hata, K., Yoshikawa, T., Sugiura, Y., and Ueda, M. Tissue-engineered bone using mesenchymal stem cells and a biodegradable scaffold. J Craniofac Sug 13, 231,2002.

[Non-patent document 9] Yamada, Y., Boo, J.S., Ozawa, R., Nagasaka, T., Okazaki, Y., Hata, K., and Ueda, M. Bone regeneration following injection of mesenchymal stem cells and fibrin glue with a biodegradable scaffold. J Cranio-Maxill Sug 31, 27, 2003.

[Non-patent document 10] Rosenberg, M., and Mortenson, W. Considerations in the corticosteroid treatment of bone cysts. J Pediatr Orthop 9, 240, 1989.

[Non-patent document 11] Schlag, G., and Redl, H. The influence of fibrin sealant on demineralized bone matrix-dependent osteoinduction: A quantitative and qualitative study in rats. Clin Orthop 238, 282, 1989.

[Non-patent document 12] Bowen-Pope, D.F., Vogel, A., and Ross, R. Production of platelet-derived growth factor-like molecules and reduced expression of platelet-derived growth factor receptors accompany transformation by a wide spectrum of agents. Pnas 81, 2396, 1984.

[Non-patent document 13] Roberts, A.B., and Spron, M.B. Physiological actions and clinical applications of transforming growth factor-beta (TGF-beta). Growth factors 8, 1, 1993.

[Non-patent document 14] Antonaides, H.N., and Williams, L.H. Human platelet-derived growth factors: Structure and functions. Federation Proc 42, 2630, 1983.

[Non-patent document 15] Yamada, Y. et al., Autogeneous Injectable Bone for Regeneration with Mesenchimal Stem Cells and Platelete-Rich Plasma:Tissu-Engineeered Bone Regeneration, TISSUE ENGINEERING, Volum 10, Number 5/6, 2004, pp955-964

[DISCLOSURE OF THE INVENTION]

[Problems to be Solved by the Invention]

As mentioned above, regenerations of the bone tissue by various techniques have been attempted. In order to achieve clinical application or practical use of the regenerated bone tissue, future problems are as follows: (1) to provide a composition for forming bone tissue having a high treatment effect with a simple and easy operation; (2) to provide a composition for forming bone tissue capable of obtaining a stable treatment effect; (3) to provide a composition for forming bone tissue with more safety, and the like.

For the composition for forming bone tissue, cells having a bone tissue forming ability are generally used. Cells having a bone tissue forming ability can be obtained by inducing differentiation of mesenchymal stem cells. Then, in order to prepare cells having a bone tissue forming ability efficiently and stably, it is necessary to collect mesenchymal stem cells from a biological sample such as bone marrow with high efficiency and reproducibility. To date, as a method for collecting mesenchymal stem cells, some methods including a method for collecting cells by using FACS (see for example, non-patent document 4) have been proposed. Above all, the method for collecting the cells by using FACS has been used by many researchers because the mesenchymal stem cells can be collected in a state in which the cells contain few other cells. However, there are many problems indicated, for example, the proliferation rate of the collected cells is low, operation is complicated, and the like. Methods other than the method using FACS have also many points to be improved that, for example, a complicated operation is needed and an efficient collection is difficult, and the like. The present invention addresses the problems discussed above, and has one object to provide a method for preparing cells having a bone tissue forming ability efficiently and stably with a simple and easy operation. Another object of the present invention is to provide a method for preparing a composition for forming bone tissue having high safety and an excellent treatment effect with a simple and easy operation.

[Means to Solve the Problems]

Firstly, the present inventors have investigated repeatedly in order to establish a method for preparing cells having a bone tissue forming ability efficiently and stably. As a result, it has been clear that when bone marrow is diluted and cultured, mesenchymal stem cells that are later used for cells for forming bone tissue can be proliferated with high efficiency and reproducibility. Furthermore, according to the commonsense, it is thought better that the dilution ratio of the bone marrow at the time of primary culture is lower. Surprisingly, however, even when the dilution ratio is increased, efficient cell proliferation was observed. Thus, the present inventors have reached to find a technique for supplying cells having a bone tissue forming ability efficiently and stably in an extremely simple and easy manner.

After the above-mentioned results are obtained, the present inventors have further investigated repeatedly for establishing a method for preparing a new composition for forming bone tissue in order to resolve the above-mentioned problems. Firstly, considering the safety and regeneration effect of bone tissue, and the like, as the components to be combined with cells, platelet-rich plasma as well as thrombin and calcium ions necessary for gelation of the platelet-rich plasma are employed. Subsequently, the present inventors have thought that the state of gelation and the treatment effect are closely related to each other, so that the present inventors have investigated the relation between the mixing ratio of the components and the state of gelation as well as the relation between the state of gelation and the treatment effect in detail. As a result, it has been shown that by mixing the components at a predetermined mixing ratio and by mixing air at a predetermined ratio, a gelation-state composition having a high treating effect and a good operation property can be obtained.

The present invention has completed based on the above-mentioned results and provides the following configuration. That is to say, the present invention provides a method for preparing cells for forming bone tissue, and the method includes the following steps (1) to (4).

(1) plating and culturing bone marrow separated from a living body in a culture flask so that it is diluted 2 fold to 2000 fold.
(2) removing a suspended component and then culturing remaining adherent cells;
(3) inducing differentiation of proliferated cells into osseous cells; and
(4) recovering the cells.

Further, the present invention provides a method for preparing a composition for forming bone tissue, the method includes the following steps (i) to (iii):

(i) providing a thrombin solution containing 100 U/ml to 10000 U/ml of thrombin;
(ii) preparing platelet-rich plasma (PRP) from blood separated from a living body; and
(iii) mixing the thrombin solution provided in the step (i), the platelet-rich plasma prepared in the step (ii), cells for forming bone tissue prepared by the method in accordance with the present invention, and air at a following mixing ratio (based on the volume ratio) in the presence of calcium ions and gelating the mixture.

The mixing ratio of the thrombin solution : a total amount of the platelet-rich plasma and the cells for forming bone tissue : air is 1 : 3 to 7 : 0.1 to 5.0.

In one embodiment of the present invention, the thrombin solution is a 5% to 25% calcium chloride solution containing 100 U/ml to 10000 U/ml of thrombin.

In one preferable embodiment of the present invention, platelet-rich plasma having the concentration rate of platelet ranging from about 150% to about 1500% is used.

In one embodiment of the present invention, the composition for forming bone tissue contains about $1.0 \times 10^5$ to about $1.0 \times 10^8$ cells/ml of cells for forming bone tissue.

As another aspect of the present invention, a method for reconstructing bone tissue is provided. The method includes infusing, embedding, packing or applying the composition for forming bone tissue obtained by the preparation method in accordance with the present invention, to a bone tissue defect portion.

[BRIEF DESCRIPTION OF THE DRAWINGS]

Fig. 1 is a flowchart showing a method for preparing cells for forming bone tissue.

Fig. 2 are views for comparing states of compositions obtained by mixing components (thrombin solution, cells for forming bone tissue and platelet-rich plasma, and air) with various mixing ratios. In numeric values in this figure represent the mixing ratios (thrombin solution : cell suspension (total amount of cells for forming bone tissue and platelet-rich plasma) : air).

[BEST MODE OF CARRYING OUT THE INVENTION]

The first aspect of the present invention relates to a method for preparing cells for forming bone tissue. The term "cells for forming bone tissue" herein denotes cells used for reconstruction (regeneration) of bone tissue and are characterized by having a bone tissue forming ability. In the present invention, cells for forming bone tissue can be obtained from the mesenchymal stem cell (undifferentiated mesenchymal stem cell) existing in bone marrow. Note here that a mesenchymal stem cell (that is, a cell for forming bone tissue) which gains a differentiation potency to the osseous cell is also referred to as an "osseous differentiation potency gaining cell."
Furthermore, the term "bone tissue" herein is used to have a broad meaning and includes bone tissue of various sites (for example, periodontal tissue).
The preparation method of the present invention includes steps of: (1) diluting bone marrow and plating it; (2) selectively culturing adherent cells; (3) inducing differentiation to osseous cells; and (4) recovering the cells.
Hereinafter, each step is described, respectively.

(1) Step of diluting bone marrow and plating it

In this step, the bone marrow separated from a living body is plated in a culture flask so that it is diluted 2-fold to 2000-fold and then it is cultured. As mentioned in the below-mentioned Example, when the bone marrow was diluted 2-fold to 500-fold and then initial culture was carried out, excellent cell proliferation was observed. According to the commonsense, it is thought that it is more preferable that the dilution ratio of the bone marrow is lower. Surprisingly, however, even when the dilution ratio was high, excellent cell proliferation was observed. Even in the conditions where the bone marrow was diluted 500-fold, extremely high cell proliferation was observed. From this result, it has been clear that the above-mentioned range of the dilution ratio is an effective dilution ratio at the initial culture. Similarly, it is predicted that higher dilution ratio can be employed as an effective condition. Note here that when the dilution ratio of the bone marrow before initial culture is high, it is advantageous in terms of the culture efficiency that the number of passages necessary for obtaining a predetermined number of cells is reduced and the culture time is reduced.
The bone marrow (mesenchymal stem cell) can be collected from the ilium, jawbone, umbilical cord blood, fat, peripheral blood, and the like, of a donor by using, for example, a syringe. However, it is particularly preferable that the bone marrow is collected from the ilium because the collection is relatively easy and burden to a donor is small, and the like.
From the viewpoint of toxicity or immunological rejection, it is preferable to use the bone marrow collected (separated) from a recipient him/herself (that is to say, a subject to whom the cells for forming bone tissue obtained by the method of the present invention is applied). However, allogeneic bone marrow may be used.
In order to prevent coagulation, in general, the collected bone marrow is brought into contact with an anticoagulant agent (heparin, sodium citrate, or the like). For example, bone marrow can be brought into contact with an anticoagulant agent by collecting the bone marrow with the use of a heparin-coated syringe. Furthermore, an anticoagulant agent may be added to the collected bone marrow.
For dilution, a culture medium is generally used. As the culture medium, for example, MSCBM (Mesenchymal Stem Cell Basal Medium) or a product obtained by adding a mesenchymal stem cell growth medium additive factor (MSCGM) to MSCBM, or DMEM can be preferably used. MSCBM and MSCGM are available from TAKARA BIO INC. (Otsu, Japan) and Sanko Junyaku Co., Ltd. (Tokyo, Japan), etc. Similarly, DMEM is available from GIBCO and others. It is preferable that a component (growth auxiliary agent) for promoting the growth of mesenchymal stem cells is added into the culture medium. As the growth auxiliary agent, serum (for example, FBS, autologous serum), L-glutamine, a penicillin-streptomycin mixture, and the like, are generally used.
The bone marrow can be diluted in several batches. In this case, each dilution ratio is adjusted so that the final dilution ratio falls in the above-mentioned range. For example, the bone marrow separated from a living body is firstly diluted two-fold, subsequently, diluted five-fold so that the bone marrow is finally diluted ten-fold. The dilution ratio in each dilution is not particularly limited. However, it is preferable that the dilution ratio of the latter dilution operation is larger than that of the former dilution operation as in this example. It is not necessary to use the same dilution fluid (usually, culture medium) for each dilution operation. For example, the first dilution may be carried out with the use of MSCBM (or DMEM, α-MEM, β-MEM, or the like) and the second dilution may be carried out with the use of MSCBM containing 10% FBS (fetal bovine serum). At the time of dilution, an anticoagulant agent may be added thereto. When the dilution is carried out in a plurality of batches, an anticoagulant agent may be added in a part of the dilution operation. When dilution is carried out with the use of an anticoagulant-containing fluid, the addition of the anticoagulant agent can be carried out simultaneously with the dilution.

As the culture flask, for example, a flask, a schale, and the like, can be used preferably.

The bone marrow diluted as mentioned above is cultured in a routine procedure. The suitable conditions for growth and proliferation of the mesenchymal stem cells are known to persons skilled in the art. For example, culture can be carried out in the following conditions: MSCBM containing 10% FBS is used and culture is carried out in the presence of 5% $CO_2$, in the wetting atmosphere, and at 37°C.

(2) Step of selectively culturing adherent cells

In this step, firstly, adherent cells are selected. The adherent cells can be selected by removing suspended components. Suspended cells can be removed easily by replacing a medium with a new one. Specifically, a part of or substantially all the medium is removed by sucking, and subsequently, a new medium is poured into a culture flask. Thus, a part of or substantially all the medium is replaced. This replacement of media may be repeated a plurality of times. In order to wash and remove the suspended components sufficiently, it is preferable that the replacement of media is carried out three to four times per week.

Adherent cells having adhesiveness (cells attached to a culture flask), which remain after removing suspended components, are further cultured. The culture herein can be carried out under the same conditions as those in step (1). During culture, the culture medium is appropriately replaced with a new one. For example, the culture medium is replaced with a new one every three days.

At the stage in which cells are proliferated to some extent, passage culture (expansion culture) may be carried out. For example, when cells are subconfluent (in a state in which about 70% of the surface of the culture vessel is occupied by cells) by visual observation, cells are peeled off from the culture flask and recovered. Then, they are plated in a culture flask filled with a culture medium. Passage culture may be repeated. For example, passage culture is carried out once to three times so that cells are proliferated to the necessary number of cells (for example, about $1 \times 10^7$ cells/ml). Note here that cells can be peeled off from the flask by a routine method such as treatment with trypsin.

(3) Step of inducing differentiation to osseous cells

In this step, proliferated cells are subjected to induction treatment so as to differentiate into osseous cells. A method for inducing differentiation of an undifferentiated mesenchymal stem cell into an osseous cell is well known. In a typical technique, the differentiation to an osseous cell is promoted by adding three kinds of additive agents, that is, dexamethasone (Dex), β-sodium glycerophosphate (β-GP), and L- ascorbic acid 2-phosphate (AsAP) into a culture medium (or replacing a medium with a medium containing such additive agents (replacing a medium with a bone inducing medium)). As the addition amount of such additive agents, for example, dexamethasone is about 10 mM, β-sodium glycerophosphate is about $10^{-8}$M, and L-ascorbic acid 2-phosphate is about 0.05 mM. During differentiation-inducing time, the medium is appropriately replaced with a new one. For example, the medium is replaced with a new one every three days. Culture for inducing differentiation continues for, for example, one day to 14 days.

(4) Step of recovering cells

In this step, mesenchymal stem cells gaining a differentiation potency to osseous cells as a result of the step (3) (in this specification, referred to as "osseous differentiation potency gaining cells" or "cells having a bone tissue forming ability") are recovered. After cells are peeled off from a culture flask by treatment with trypsin or the like, the cells are subjected to centrifugation, and thereby cells can be recovered.

Next, another aspect of the present invention is described. This aspect relates to one embodiment of the use of the cells for forming bone tissue prepared by the above-mentioned method of the present invention. That is to say, this aspect provides a method for preparing a composition for forming bone tissue.

The preparing method of the present invention includes (i) a step of providing a thrombin solution; (ii), a step of preparing platelet-rich plasma (PRP); and (iii) a step of mixing each component and gelating the mixture. Hereinafter, each step is described, respectively.

(i) Step of providing thrombin solution

In this step, a solution containing a predetermined amount of thrombin is provided. The concentration of thrombin in the thrombin solution is not particularly limited but made to be a concentration at which an appropriate gelation can be achieved in the below-mentioned step (iii). For example, the concentration of the thrombin solution is determined so that the thrombin solution contains 100 U/ml to 10000 U/ml of thrombin. Preferably, the thrombin concentration is made to be about 1000 U/ml. From the viewpoint of safety and immunological rejection, human thrombin is preferably used. As the human thrombin, for example, Thrombin-YOSHITOMI (registered trademark) can be used. Alternatively, human thrombin prepared from the autologous blood may be used. By acting thrombin in the presence of calcium ions, fibrin is generated from fibrinogen in platelet-rich plasma (PRP) and coagulated (gelated).

Therefore, if a thrombin solution containing a calcium ion is provided, when the thrombin solution and PRP are mixed (step (iii)), it is not necessary to add calcium ions. For example, it is preferable that a thrombin solution is prepared as a 5% to 25% calcium chloride solution. It is further preferable to use a thrombin solution prepared as an about 10% calcium chloride solution.

(ii) Step of preparing platelet-rich plasma (PRP)

In this step, platelet-rich plasma (PRP) is prepared from blood separated from a living body. Herein, "platelet-rich plasma" or PRP refers to plasma containing abundance of platelets. In other words, it refers to plasma containing concentrated platelet. PRP can be prepared by subjecting blood collected to centrifugation in accordance with, for example, the method by Whitman et al. (Dean H. Whitman et al.: J Oral Maxillofac Surg, 55, 1294-1299 (1997)). PRP is known to include an abundance of growth factors such as Platelet-derived Growth Factor (PDGF), Transforming growth factor β1 (TGF-β1), Transforming growth factor β2 (TGF-β2), and the like (Jarry J. Peterson: Oral surg Oral Med Oral Pathol Oral Radiol Endod, 85, 638-646 (1998)).

PRP can be prepared in accordance with the Nisseki PC (platelet concentrated) collection method. Specific example of the method for preparing PRP is described hereinafter. Firstly, an anticoagulant agent such as sodium citrate is added to the collected blood and the collected blood is stood still for a predetermined time at room temperature, followed by subjecting it to centrifugation under conditions in which blood cells and buffy coat are separated (for example, at about 1,100 rpm for about 10 minutes). Thus, the blood is divided into two layers. The upper layer is collected and then the remaining blood is further centrifuged at about 2,500 rpm for about 10 minutes. The resultant fragments (Platelet-rich Plasma: PRP) are collected. The method for preparing PRP is not limited to this alone. PRP can be prepared by a method that has been modified if necessary.

From the viewpoint of toxicity and immunological rejection, it is preferable that PRP is prepared by using the blood of a recipient him/herself (that is to say, a subject to whom the cell for forming bone tissue obtained by the present invention is applied). However, PRP may be prepared from allogeneic blood.

The number of platelets contained in PRP (concentration rate of platelet) is not generally defined. The plasma containing platelets that are about 150 % to about 1500 % more than those of the collected blood may be defined as PRP of the present invention.

The "platelet concentration rate" of PRP of the present invention is expressed by the following equation.

$$\text{platelet concentration rate (\%)} = \text{(average number of platelets in PRP)} / \text{(average number of platelets in whole blood as a starting material)} \times 100$$

Therefore, when for example, the average number of platelets in PRP is 1,000,000 and the average number of platelets in whole blood is 300,000, the platelet concentration rate (%) is about 333%. As a result of the investigation by the present inventors, it has been clear that the platelet concentration rate of PRP has a relation with respect to the regeneration effect of the bone (or periodontal) tissue. Therefore, in order to obtain the higher regeneration effect, it is preferable to use PRP having a platelet concentration rate in the range from about 150% to about 1500% (generally corresponding to about 240,000 cells/μL to about 6,150,000 cells/μL when converted into the average number of platelets). More preferably, it is preferable to use PRP having a platelet concentration rate in the range from about 300% to about 700% (generally corresponding to about 480,000 cells/μL to about 2,870,000 cells/μL when converted into the average number of platelets).

By appropriately adjusting the conditions of the centrifugation when PRP is prepared, it is possible to obtain PRP with a desired platelet concentration rate. For example, when the two-stage centrifugation as mentioned above is carried out and when the first centrifugation is carried out under the conditions of about 500 rpm to about 1500 rpm (for example, 1,100 rpm) for about 5 minutes to about 15 minutes (for example, for about 5 minutes) and the second centrifugation is carried out under the conditions of about 2000 rpm to about 5000 rpm (for example, 2,500 rpm) for about 5 minutes to about 15 minutes (for example, for about 5 minutes), it is possible to obtain PRP having the platelet concentration rate ranging from about 300% to about 700%. It is predicted that the platelet concentration rate of finally obtained PRP varies due to the difference in the blood as the starting material and instruments to be used even if the treatment is carried out under the same condition. The person skilled in the art can find conditions for preparing PRP with a desired platelet concentration rate by modifying the conditions based on the platelet concentration rate of the obtained PRP while considering the above-mentioned conditions.

Note here that the measurement of the platelet concentration of PRP can be carried out in accordance with a routine procedure (for example, by using commercially available Sysmex XE-21 00 (Sysmex, Tokyo, Japan) as shown in Example).

Platelet concentration rate of PRP used for constructing the composition of the present invention is as mentioned above. On the other hand, the platelet concentration of the final composition (the composition of the present invention) varies depending upon the platelet concentration rate of PRP and the using ratio of PRP and other components combined with PRP (that is to say, cells having a bone tissue forming ability, and the like). However, the platelet concentration is for example, about 240,000 cells/$\mu$L to about 6,150,000 cells/$\mu$L, and preferably about 480,000 cells/$\mu$L to about 2,870,000 cells/$\mu$L. By containing platelets in such a concentration, an excellent effect of regenerating bone (or periodontal) tissue is obtained. Note here that for example, by using PRP having the platelet concentration rate ranging from about 300% to about 700%, it is possible to adjust the platelet concentration of the final composition to be about 480,000 cells/$\mu$L to about 2,870,000 cells/$\mu$L.

(iii) Step of mixing components and gelating mixture

In this step, the thrombin solution provided in the step (i), the platelet-rich plasma prepared in the step (ii) and cells for forming bone tissue prepared by the method described in the present invention are mixed in the presence of calcium ions and the mixture is gelated. In the present invention, when these components are mixed, air is mixed at a predetermined ratio. When air is mixed, the gelation state (fluidity) can be adjusted. Furthermore, when the composition for forming bone tissue in which air is mixed is transplanted into a living body, with the appropriate amount of air existing in the vicinity of the composition, an environment suitable for cells having a bone tissue forming ability in the composition to survive and grow can be made. Thus, an excellent tissue regeneration effect can be expected.

In the present invention, components are mixed at the following mixing ratio (based on the volume) and the mixture is gelated.

(a) thrombin solution : total amount of platelet-rich plasma and cells for forming bone tissue : air = 1 : 3 to 7 : 0.1 to 5.0
By mixing the components with the above-mentioned mixing ratio, it is possible to obtain a gel-state composition having an appropriate fluidity from the viewpoint of an operation property and fixity after transplantation as well as exerting an excellent regeneration effect (treatment effect).
Herein, the larger the mixing ratio of the total amount of the platelet-rich plasma and cells for forming bone tissue becomes and/or the larger the mixing ratio of air becomes, the lower the fluidity of the obtained gel-state composition becomes. That is to say, by manipulating the mixing ratio of (a), the fluidity of the obtained gel-state composition can be adjusted. Specifically, if a composition with relatively low fluidity is needed, among the above-mentioned range of the mixing ratios, the mixing ratio, in which the total amount of platelet-rich plasma and cells for forming bone tissue (and/or the mixing ratio of air) is small, may be employed. If a composition with relatively high fluidity is needed, among the above-mentioned range of the mixing ratios, the mixing ratio, in which the total amount of platelet-rich plasma and cells for forming bone tissue (and/or the mixing ratio of air) is large, may be employed.
Note here that since the thrombin solution, the platelet-rich plasma and the cells for forming bone tissue are components originated in a living body, the characteristics may vary to some extent due to the difference in the collection source and the like, and this is thought to affect the gelation state. However, when according to the investigation results to date by the present inventors, the components are mixed in the above-mentioned range of mixing ratio, it is confirmed that a gelation-state composition for forming bone tissue having an excellent property as mentioned above can be obtained.

In one preferable embodiment of the present invention, the mixing ratio of each component is as follows.

(al) thrombin solution : total amount of platelet-rich plasma and cells for forming bone tissue : air = 1 : 4 to 6 : 0.3 to 3.0
By employing this mixing ratio, a gelation-state composition for regenerating bone having a desired fluidity can be prepared more reliably.
The concrete mixing ratio of each component is shown as follows.
thrombin solution : total amount of platelet-rich plasma and cells for forming bone tissue : air = 1 : 4 : 1.0

thrombin solution : total amount of platelet-rich plasma and cells for forming bone tissue : air = 1 : 5 : 1.0
thrombin solution : total amount of platelet-rich plasma and cells for forming bone tissue : air = 1 : 6 : 1.0

By mixing each component at the above-mentioned ratio, typically, a composition containing about $1.0 \times 10^5$ to about $1.0 \times 10^8$ cells/ml of cells for forming bone tissue can be obtained. According to such a composition, when it is applied to a bone tissue defect portion, an excellent tissue regeneration effect can be expected.

As explained above, in the method in accordance with the present invention, a composition for forming bone tissue is obtained by mixing a thrombin solution, platelet-rich plasma, cells for forming bone tissue and air. However, it is not intended to exclude an additional use of other components as long as the obtained effect of the composition for regenerating bone tissue (bone tissue regeneration effect) is maintained. Components to be additionally used in the method of the present invention is exemplified as follows.

(Inorganic bioabsorbable material and organic bioabsorbable material)

The kinds of the inorganic bioabsorbable material are not particularly limited, but it is possible to use a material selected from the group consisting of β-tricalcium phosphate (β-TCP), α-tricalcium phosphate (α-TCP), tetracalcium phosphate, octacalcium phosphate, and amorphous calcium phosphate. These materials can be used singly. In addition, the combination of arbitrarily selected two or more materials may be used. Preferably, it is possible to use either β-TCP or α-TCP and the combination of them at arbitrary ratio may be used. More preferably, β-TCP is used as an inorganic bioabsorbable material.
The inorganic bioabsorbable material can be obtained by a well-known method. Furthermore, commercially available inorganic bioabsorbable material can be also used. As the β-TCP, for example, one manufactured by OLYMPUS CORPORATION can be used.

It is preferable that the inorganic bioabsorbable material has a powdery form having a particle diameter such that the composition of the present invention has a fluidity when it is used.
The powdery inorganic bioabsorbable material can be prepared by breaking and crushing an inorganic bioabsorbable material that has been processed so that it has an appropriate size. It is preferable that the average particle diameter of the inorganic bioabsorbable material is 0.5 μm to 50 μm. It is further preferable that the inorganic bioabsorbable material having the average particle diameter of 0.5 μm to 10 μm is used. It is yet further preferable that the inorganic bioabsorbable material having the average particle diameter of 1 μm to 5μ m is used. It is also possible to use the combination of a plurality of inorganic bioabsorbable materials having different particle diameters.
It is preferable that the content of the inorganic bioabsorbable material is 30 wt.% to 75 wt.% with respect to the entire composition.
Note here that the fluidity of the composition of the present invention can be adjusted by the particle diameter and content of the inorganic bioabsorbable material and by appropriately adjusting the both, a desired fluidity can be obtained. Furthermore, when a thickener mentioned below is added, the fluidity can be also adjusted by the addition amount of the thickener.

As the organic bioabsorbable material, collagen, fibrinogen (for example, Bolheal (registered trademark)), and the like, can be used.

(Gelation material)

For example, the composition of the present invention can be constructed by adding thrombin and calcium chloride. When such materials are added, thrombin acts on the fibrinogen in the PRP, so that fibrin is generated. Then, due to the coagulation action of fibrin, viscosity is increased. The kinds of the gelation materials are not particularly limited and a material that increases the viscosity by acting the component in the PRP as described above, or a material having a thickening effect by itself can be appropriately selected and used.
Furthermore, in addition to the above-mentioned gelation materials, a second gelation material, which acts after application (after transplantation) so as to change the fluidity (viscosity) of the composition of the present invention, can be used. With such a configuration, the composition is easily transplanted because it has an appropriate fluidity when it is used, and the composition has an improved fixation at the application site because it has an increased viscosity after application. Thus, the bone tissue or the periodontal tissue can be repaired or regenerated efficiently. Furthermore, it is not necessary to shape the material in a shape of the site to be applied in advance, thus increasing the versatility.
As the gelation material, a material having a high biocompatibility is preferably used. In addition to the above-mentioned examples, collagen or fibrin glue, or the like, can be used. Various kinds of collagen can be selected and used. However, it is preferable to employ collagen suitable for application object of the composition (tissue to which the composition is applied) in accordance with the present invention. When the object is to regenerate the bone tissue, for example, type I collagen can be used. It is preferable that the collagen to be used has a solubility (acid soluble collagen, alkali soluble collagen, enzyme soluble collagen, and the like).

(Thickener)

It is also possible to adjust the fluidity of the composition of the present invention by adding a thickener. As the thickener, thickening polysaccharides such as sodium alginate, glycerine, vaseline, or the like, can be used. From the viewpoint of safety and/or bone forming ability, it is preferable to use a thickener having a high biocompatibility and having a bioabsorbable property or a biodegradability. By adding glycerine or the like, an antifreezing effect can be obtained.

(Solvent)

The composition of the present invention may include an aqueous solvent. An example of the aqueous solvent can include sterile water, a physiological saline solution, a buffer solution such as a phosphate solution, and the like.

(Others)

The composition of the present invention may include a stabilizer, a preservative, a pH regulator, and the like, in addition to the above-mentioned components. Furthermore, the composition may also include a growth factor, in particular, a bone inducing factor (BMP).
The composition for forming bone tissue obtained by the preparation method of the present invention is used for repairing or regenerating (reconstructing) bone tissue. That is to say, the composition for forming bone tissue of the present invention can be used as a bone regeneration material instead of an autologous bone or ceramics materials (β-TCP and hydroxyapatite) and Bio-Oss (registered trademark) that have been conventionally used in a maxillary sinus lift technique, periodontal disease treatment, a bone regeneration technique, and the like. The composition for forming bone tissue of the present invention is used, for example, in a state in which it is infused, embedded, packed, applied or the like for a site that needs repairing of bone tissue (i.e., bone tissue defect portion). An example of the indicated case may include periodontal disease, bone regeneration for implant, a gnathoschisis site, a bone extending site, after odontectomy, a cyst extraction site, a tumor excised site, an artificial joint site, osteoporosis, and the like.

[Example]

1. Investigation of method for isolating cell having bone tissue forming ability

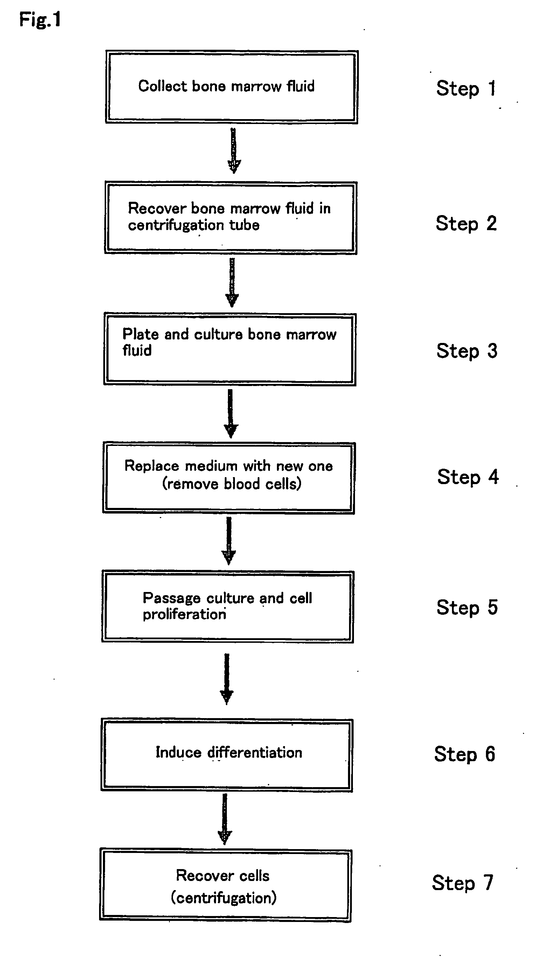

In order to establish a method for efficiently and stably preparing cells having a bone tissue forming ability using bone marrow as a starting material, the following experiment was carried out. Firstly, the present inventors thought that a technique that is as simple as possible is necessary and decided to prepare cells having a bone tissue forming ability by way of a series of operations including (1) collecting (isolating) bone marrow fluid; (2) plating and culturing the bone marrow fluid; (3) selecting adherent cells; (4) inducing differentiation of the cells to osseous cells; and (5) recovering the cells. In the operation (2), the present inventors focused on the dilution ratio at the time of plating bone marrow fluid, and examined the relation between the dilution ratio and the number of the finally obtained cells. Note here that the operation procedure in each step was carried out as follows (see Fig. 1).

<Operation procedure>

(1) Collection of bone marrow fluid (Step 1 in Fig. 1)

The ilium bone marrow fluid (5 to 50 ml) was collected from the ilium of a patient by using a bone marrow puncture needle into a 10 ml-syringe (TERUMO CORPORATION) coated with 0.5 mL of heparin (1000 U/ ml, MOCHIDA PHARMACEUTICAL CO., LTD.).

(2) Plate and culture of bone marrow fluid (Step 2 of Fig. 1)

The collected bone marrow fluid was transferred to a centrifugation tube including 0.5 mL of heparin (1000 U/ ml, MOCHIDA PHARMACEUTICAL CO., LTD.) and 10 mL of a growth medium (a medium obtained by adding 10% FBS or autologous serum, L-glutamic acid, and penicillin / streptomycin to MSCBM (or DMEM, α-MEM, β-MEM, or the like), Cambrex) (first dilution: two fold dilution). the bone marrow fluid was diluted with a medium (MSCBM + 10% FBS) so as to reach a predetermined dilution ratio (second dilution: 1 to 250 fold dilution), followed by plating it in a flask (80

$cm^2$, Greiner labortechnik Germany). The flask was transferred to the inside of an incubator and cultured under conditions of 5% $CO_2$, humidified atmosphere at 37°C).

(3) Selection of adherent cell (Steps 3 to 5 in Fig. 1)

In order to remove blood cell-based cells contained in the culture medium and to select and culture adherent cells, 24 hours after the plating of bone marrow cells, the culture medium was replaced with a new one. Thereafter, the cells were continued to be cultured for about 7 to 14 days so that the cells were subconfluent in the culture medium. During this time, the culture medium was replaced with a new one every three days. Subsequently, cells were peeled off by using 0.05% trypsin, and they were plated so that the cells were two to four times with respect to the area ratio so as to carry out passage culture (expansion culture).

(4) Inducing differentiation to osseous cell (Step 6 in Fig. 1)

Firstly, cells were cultured for about six days from the passage culture. Then, it was confirmed that the cells reached subconfluent and, β-sodium glycerophosphate (Sigma Chemical Co., St Louis, US), dexamethasone (Sigma Chemical Co., St Louis, USA), and L-ascorbic acid 2-phosphate (Sigma Chemical Co., St Louis, USA) were added to the culture medium so that the concentrations became 10 mM, $10^{-8}$ M and 0.05 mM, respectively, and culture (in a bone inducing medium) was continued. The medium was replaced with a new one every three days. Every time the culture was replaced with a culture medium to which β-sodium glycerophosphate, dexamethasone and L-ascorbic acid 2-phosphate were added as mentioned above.

(5) Recovering cells (Step 7 in Fig. 1)

The medium was removed from the flask with a small amount of the medium remained. By using trypsin / EDTA, cells were peeled off from the flask. The peeled cells were transferred to a centrifugation tube together with the medium and subjected to centrifugation (1500 rpm, 5 min). The supernatant was sucked and the cell components were recovered.

<Results>

In a group in which the second dilution was carried out with the dilution ratio of 1 to 250 fold (the dilution ratio of the total of the first and second dilution was 2 fold to 500 fold), it was revealed that the number of adherent cells obtained as a result of the operation (3) was increased remarkably. It was confirmed that the cells obtained as a result of the series of operations (1) to (5) were differentiated to osseous cells by using an alkaline phosphatase (ALP) staining technique by alkaline phosphatase using p-nitrophenylphosphatase, respectively.
From the above-mentioned results, it was revealed that when bone marrow fluid that had been diluted 2 to 500 fold was plated and cultured and then selection operation of adherent cells was carried out, adherent cells (mesenchymal stem cells) were able to be selected efficiently with high recovering rate and the number of the finally obtained cells (cells having a bone tissue forming ability) was increased.
By employing the effective dilution ratio, which has been cleared in this way, and by using bone marrow collected from other patients, a plurality of additional tests were carried out. In any cases, the number of the finally obtained cells (cells having a bone tissue forming ability) was not so different from each other, and efficient preparation could be carried out. From this result, it was confirmed that by employing the above-mentioned range of the dilution ratio, the cells having a bone tissue forming ability were able to be supplied stably.

2. Investigation of gelation method of composition for forming bone tissue

In order to establish a method for preparing a composition for forming bone tissue that has an excellent operation property and is excellent in a treatment effect, the present inventors carried out the following experiment. Firstly, by considering the reports to date and the experience of the present inventors, as the components to be contained in the composition, the present inventors selected cells having a bone tissue forming ability and platelet-rich plasma (PRP). Then, for gelation, thrombin was used. Thus, the present inventors used roughly divided three components, that is, cells, PRP, and thrombin so as to prepare a composition for forming bone tissue and investigated the relation between the mixing ratio of the components and the gelation state by the following method.

<Materials and methods>

(1) Provision and preparation of each component

Human thrombin (YOSHITOMI) was dissolved in a 10% calcium chloride solution and a 1000 U/ml of thrombin solution was provided.

Cells having a bone tissue forming ability were prepared by the same procedure as mentioned in the <Operation procedure> in the Example mentioned above. However, the dilution ratio of bone marrow (second dilution ratio) was made to be five-fold dilution.

Meanwhile, about 50 mL to about 200 mL of whole blood was collected from a patient and put into a donate blood bag together with heparin (250 U/mL) without containing preservatives. The whole blood was centrifuged at 1100 rpm for five minutes, followed by collecting yellow plasma (containing platelet and buffy coat including white blood cells) with the use of a cannula. Next, the collected sample was centrifuged twice at 2500 rpm for 5 minutes so as to pellet the platelet. Then, plasma supernatant being poor platelet plasma (PPP) and having a relatively small content of cells was removed. The obtained platelet pellet, that is, a buffy coat / plasma fraction was suspended in the remaining plasma (5 mL) so as to obtain PRP. The numbers of platelets in the PRP and the PPP were measured by using Sysmex XE-21 00 (Sysmex, Tokyo, Japan). As a result, the total number of platelets was 219,000 on average (in the range from about 160,000 to about 410,000). On the other hand, the number of platelets in the PRP was 1,136,000 on average (in the range from about 490,000 to about 2,130,000). From these measurement results, it was able to be confirmed that the platelet was separated and it was revealed that the concentration rate in the PRP was 485% (based on the number of total blood (100%)). The PRP was stored in a general shaker at room temperature before use.

(2) Mixing each component and gelating mixture

Cells having a bone tissue forming ability were suspended in PRP so as to obtain a cell suspension solution (about $1.0 \times 10^6$ to about $1.0 \times 10^8$ cells/mL). Note here that the number of cells is preferably about $1.0 \times 10^7$ cells/mL. This cell suspension, a thrombin solution and air were mixed with each other at a mixing ratio (based on the volume) and by the method mentioned below. Then, the gelation states were compared.

(Mixing-ratio)

Thrombin solution cell suspension : air

| | |
|---|---|
| Condition 1 | 1 : 1 : 1 |
| Condition 2 | 1 : 2 : 1 |
| Condition 3 | 1 : 3 : 1 |
| Condition 4 | 1 : 4 : 1 |
| Condition 5 | 1 : 5 : 1 |
| Condition 6 | 1 : 6 : 1 |
| Condition 7 | 1 : 7 : 1 |

| | |
|---|---|
| Condition 8 | 1 : 8 : 1 |
| Condition 9 | 1 : 10 : 1 |
| Condition 10 | 1 : 12 : 1 |
| Condition 11 | 1 : 20 : 1 |
| Condition 12 | 1 : 50 : 1 |
| Condition 13 | 1 : 100 : 1 |

(Mixing method)

A predetermined amount of cell suspension solution and air were sucked into an injector. A predetermined amount of thrombin solution and air were sucked into another injector. The thus provided two injectors were linked to each other with the use of a three way stopcock, followed by pushing the pistons of the both injectors alternately so that contents come and go in both injectors. Thus, mixing and gelation were carried out.

(3) Transplantation experiment

An bone defect model was produced with the use of trephine bar (10 mm) in after extracting the dog mandibular bone in accordance with the method described in TISSUE ENGINEERING, Volum 10, Number 5/6, 2004, pp955-964 (Yamada, Y. et al., Autogeneous Injectable Bone for Regeneration with Mesenchimal Stem Cells and Platelete-Rich Plasma:Tissu-Engineeered Bone Regeneration). The gelated composition for forming bone tissue was packed in the bone defect portion and the bone forming ability (tissue reconstruction effect) was investigated histologically and tissue morphologically. As comparison subjects, a test group of only defect portion and a test group in which the autologous bone was packed were provided.

<Results>

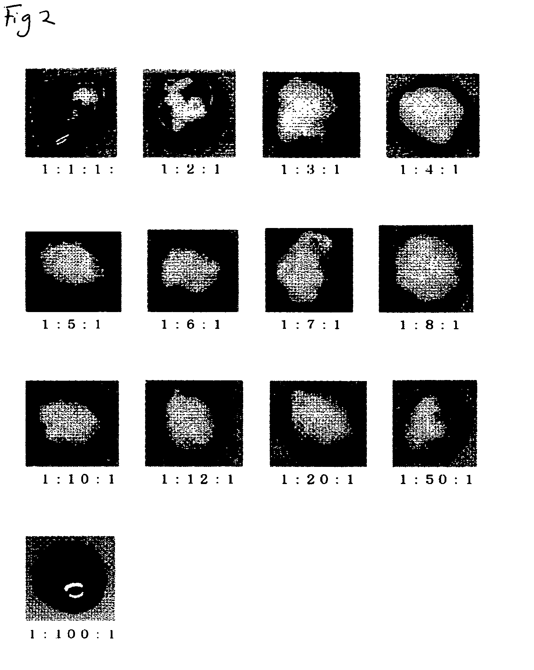

(1) The states of the compositions obtained by mixing components under the conditions are shown in Fig. 2. It has been revealed that in the mixing ratio ranging from 1 : 3 : 1 to 1 : 7 : 1 (conditions 3 to 7), a gel-state composition having suitable fluidity can be obtained that is suitable for being infused to an affected part (for example, a periodontal tissue defect portion) with the use of an injector and well retained in the affected part after infusion. In particular, the gel-state composition obtained in the mixing ratio ranging from 1 : 4 : 1 to 1 : 7 : 1 can be sucked and infused easily by using an injector. Moreover, the retained state in the affected part after infusion is extremely good. Furthermore, when the mixing ratio is set in this range, even if the state of each component is somewhat changed, it is thought that a gel-state composition having a suitable fluidity can be obtained reliably.
(2) When the composition, which had been excellently gelated, was transplanted into the bone defect portion so as to investigate the regeneration effect, in any cases, an excellent regeneration effect was observed (the regeneration effect that is the same as that of a group into which an autologous bone was transplanted). Note here that a composition whose gelation was bad, that is, for example, in a composition obtained by employing the condition 11, since the fluidity is low, it is difficult to infuse the composition into an affected part by using an injector. Furthermore, the survival with respect to the affected part is bad. On the other hand, in the compositions obtained by employing conditions 1 and 2, the fluidity is so high that survival with respect to the affected part and the retaining property in the affected part are bad.
(3) Since it is predicted that the gelation state varies depending upon the mixing amount of air, the present inventors examined the relation between the mixing amount of air and the gelation state. The results have revealed that in appropriate mixing ratios (mixing ratios of the both in the conditions 3 to 7) of the thrombin solution and the cell suspension solution, which have been shown to be appropriate in the above-mentioned experiment, when the mixing ratio of air is made to be 0.1 to 1.5 with respect to 1 of the thrombin solution, an excellent gelation state can be obtained. In particular, when the mixing amount of air is in the range from 0.3 to 3.0, a composition with substantially an equal level of fluidity can be obtained.

[Industrial applicability]

The cells for forming bone tissue or the composition for forming bone tissue prepared by the method of the present invention can be applied to various fields in which repairing and regeneration of bone tissue (including periodontal tissue) are needed. For example, the present invention can be applied to bone regeneration when an artificial dental root is transplanted in the alveolar ridge in which high bone resorption is observed. Furthermore, the present invention can be applied to regeneration of bone tissue in a bone defect portion caused by external injury and various bone diseases, and reinforcement or compensation of the bone. Furthermore, the present invention can be applied to the regeneration of alveolar bone and periodontal tissue in a defect portion of alveolar bone caused by, for example, periodontal disease. As an application method, in the case of the cells for forming bone tissue, they can be transplanted in the application site singly or in combination with other components. Furthermore, in the case of the composition for forming bone tissue, since it is in a gel state having an appropriate fluidity, it can be applied to a site to be applied in a simple and easy manner by packing, infusing, application, or the like.
The composition for forming bone tissue prepared by the method of the present invention includes a large number of growth factors having an ability for promoting proliferation or differentiation of osteogenic cells. Therefore, in the site to be applied (transplanted portion), osteogenic cells can be proliferated or differentiated efficiently and the formation of the bone tissue or the periodontal tissue can be promoted. It is thought that by using an autologous PRP, the regeneration of bone tissue or periodontal tissue can be improved in terms of quality and quantity by the above-mentioned growth factors those do not have toxicity and that is immunologically inactive. Furthermore, since the composition is in a gel state, it can be packed easily (it can be applied without opening a wounded area) by using an injector or the like. Furthermore, it is not necessary to shape the composition in accordance with the shape of the bone defect portion in advance, and the composition can be widely used. Thus, the use of the composition for forming bone tissue prepared by the method according to the present invention makes it unnecessary to collect an autologous bone and transplant it. Thus, it is possible to generate bone or periodontal tissue easily.

The present invention is not limited to the description, of the above exemplary embodiments and Examples. A variety of modifications, which are within the scopes of the following claims and which are easily achieved by a person skilled in the art, are included in the present invention.

Contents of the theses, Publication of Patent Applications, Patent Publications, and other published documents referred to in this specification are herein incorporated by reference in its entity.

**Claims**

1.   A method for preparing cells for forming bone tissue, the method comprising the following steps (1) to (4):

(1) plating and culturing bone marrow separated from a living body in a culture flask so that it is diluted 2 fold to 2000 fold;
(2) removing a suspended component and then culturing remaining adherent cells;
(3) inducing differentiation of proliferated .cells into osseous cells; and
(4) recovering the cells.

2.   A method for preparing a composition for forming bone tissue, the method comprising the following steps (i) to (iii):

(i) providing a thrombin solution containing 100 U/ml to 10000 U/ml of thrombin;
(ii) preparing platelet-rich plasma (PRP) from blood separated from a living body; and
(iii) mixing the thrombin solution provided in the step (i), the platelet-rich plasma prepared in the step (ii), cells for forming bone tissue prepared by the method according to claim 1, and air at a following mixing ratio (based on the volume) in the presence of calcium ions, and gelating the mixture,

wherein the mixing ratio of the thrombin solution : a total amount of the platelet-rich plasma and the cells for forming bone tissue : air is 1 : 3 to 7 : 0.1 to 5.0.

3.   The method according to claim 2, wherein the thrombin solution is a 5% to 25% calcium chloride solution containing 100 U/ml to 10000 U/ml of thrombin.

4.   The method according to claim 2 or 3, wherein the concentration rate of the platelet in the platelet-rich plasma is in the range from about 150% to about 1500%.

5.   The method according to any of claims 2 to 4, wherein the composition for forming bone tissue contains about $1.0 \times 10^5$ to about $1.0 \times 10^8$ cells/ml of cells for forming bone tissue.

6.   A composition for forming bone tissue obtained by the method according to any of claims 2 to 5.

7.   A method for reconstructing bone tissue, the method comprising infusing, embedding, packing or applying the composition for forming bone tissue obtained by the method according to any of claims 2 to 5 to a bone tissue defect portion.

Fig.1

| | |
|---|---|
| Collect bone marrow fluid | Step 1 |
| ↓ | |
| Recover bone marrow fluid in centrifugation tube | Step 2 |
| ↓ | |
| Plate and culture bone marrow fluid | Step 3 |
| ↓ | |
| Replace medium with new one (remove blood cells) | Step 4 |
| ↓ | |
| Passage culture and cell proliferation | Step 5 |
| ↓ | |
| Induce differentiation | Step 6 |
| ↓ | |
| Recover cells (centrifugation) | Step 7 |

Fig 2

1 : 1 : 1 :    1 : 2 : 1    1 : 3 : 1    1 : 4 : 1

1 : 5 : 1    1 : 6 : 1    1 : 7 : 1    1 : 8 : 1

1 : 10 : 1    1 : 12 : 1    1 : 20 : 1    1 : 50 : 1

1 : 100 : 1

<div align="center">

**INTERNATIONAL SEARCH REPORT**

</div>

| | International application No. |
|---|---|
| | PCT/JP2006/309548 |

A. CLASSIFICATION OF SUBJECT MATTER
*C12N5/00*(2006.01), *A61L27/00*(2006.01), *C12N5/06*(2006.01)

According to International Patent Classification (IPC) or to both national classification and IPC

B. FIELDS SEARCHED

Minimum documentation searched (classification system followed by classification symbols)
*C12N5/00*(2006.01), *A61L27/00*(2006.01), *C12N5/06*(2006.01)

Documentation searched other than minimum documentation to the extent that such documents are included in the fields searched

Electronic data base consulted during the international search (name of data base and, where practicable, search terms used)
BIOSIS/MEDLINE/WPIDS(STN), JMEDPlus(JDream2), JST7580(JDream2), JSTPlus(JDream2), Igaku·Yakugaku Yokoshu Zenbun Database

C. DOCUMENTS CONSIDERED TO BE RELEVANT

| Category* | Citation of document, with indication, where appropriate, of the relevant passages | Relevant to claim No. |
|---|---|---|
| X/Y | WO 2002/17983 A1 (Osteogenesis Co., Ltd.), 07 March, 2002 (07.03.02), Full text & AU 200180164 A | 1/2-6 |
| X/Y | WO 2003/011353 A1 (Mitsuo OCHI), 13 February, 2003 (13.02.03), Full text & EP 1419791 A1 & KR 2004017842 A & AU 2002355688 A1 & US 2004/185085 A1 & JP 2003-516583 A & CN 1537020 A | 1/2-6 |
| Y | WO 2002/40071 A1 (Osteogenesis Co., Ltd.), 23 May, 2002 (23.05.02), Full text & AU 200212739 A & JP 2002-542441 A | 2-6 |

| ☒ | Further documents are listed in the continuation of Box C. | ☐ | See patent family annex. |

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 30 June, 2006 (30.06.06) | 11 July, 2006 (11.07.06) |

| Name and mailing address of the ISA/<br>Japanese Patent Office | Authorized officer |
|---|---|
| | |
| Facsimile No. | Telephone No. |

Form PCT/ISA/210 (second sheet) (April 2005)

**INTERNATIONAL SEARCH REPORT**

International application No.

PCT/JP2006/309548

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-055237 A (Japan Science and Technology Corp.), 26 February, 2003 (26.02.03), Full text (Family: none) | 1-6 |
| A | Kitoh, H. et al., Transplantation of marrow-derived mesenchymal stem cells and platelet-rich plasma during distraction osteogenesis— a preliminary result of three cases Bone (2004), Vol.35, No.4, pages 892 to 898 | 1-6 |
| A | Yoichi YAMADA et al., "Chunyugata Baiyokotsu", CLINICAL CALCIUM (2001), Vol.12, No.2, pages 228 to 232 | 1-6 |
| A | Izumi ASAHINA, "Takesshoban Kessho (PRP) ni yoru Kotsusoshiki Saisei no Sokushin", Shikai Tenbo (2006), Vol.97, No.6, pages 1322 to 1323 | 1-6 |
| A | Tomonobu MATSUNO et al., "PRP (Platelet-rich plasma) no Bunriho to Gel-ka ni Kansuru Kento-Enshin Bunri to Enka-Calcium ni yoru Kesshoban no Kasseika-", Shiyaku Ryoho (2002), Vol.21, No.2, pages 53 to 58 | 1-6 |
| A | Yasuhiro OKAZAKI et al., "PRP o Mochiita Kotsusaisei ni kansuru Jikkenteki Kenkyu", Journal of the Japanese Stomatological Soceity (2001), Vol.50, No.6, page 571, 94. | 1-6 |

Form PCT/ISA/210 (continuation of second sheet) (April 2005)

**INTERNATIONAL SEARCH REPORT**

| International application No. |
|---|
| PCT/JP2006/309548 |

**Box No. II        Observations where certain claims were found unsearchable (Continuation of item 2 of first sheet)**

This international search report has not been established in respect of certain claims under Article 17(2)(a) for the following reasons:

1. ☒ Claims Nos.: 7
    because they relate to subject matter not required to be searched by this Authority, namely:
    Claim 1 involves [methods for treatment of the human body by therapy] and thus relates to a subject matter which this International Searching Authority is not required, under the provisions of the PCT Rule 39.1(iv), to search.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0217983 A

- WO 0240071 A

### Non-patent literature cited in the description

- **LANGER, R. ; VACANTI, J.P.** *Tissue engineering. Science,* 1993, vol. 260, 920
- **VACANTI, J.P. ; MORSE, M.A. ; SALTZMAN, W.M. ; DOMB, A.J. ; PEREZ-ATAYDE, A. ; LANGER, R.** Selective cell transplantation using bioabsorbable artificial polymers as matrices. *J Pediatr Surg,* 1988, vol. 23, 3
- **VACANTI, J.P.** Beyond transplantation. *Arch. Surg,* 1998, vol. 123, 545
- **PITTENGER, M.F. ; MACKAY, A.M. ; BECK, C.B. ; JAISWAL, R.K. ; DOUGLAS, R. ; MOSCA, J.D. ; MOORMAN, M.A. ; SIMONETTI, D.W. ; CRAIG, S. ; MARSHAK, D.R.** Multilineage potential of adult human mesenchymal stem cells. *Science,* 1999, vol. 284, 143
- **OWEN, M. ; FRIEDENSTEIN, A.J.** Stromal stem cells: Marrow derived osteogenic precursors. *Ciba Found Symp,* 1998, vol. 136, 42
- **KADIYALA, S. ; YOUNG, R.G. ; THIEDE, M.A. ; BRUDER, S.P.** Culture expanded canine mesenchymal stem cells possess osteochondrogenic potential in vivo and in vitro. *Cell Transplant,* 1997, vol. 6, 125
- **BRUDER, S.P. ; KURTH, A.A. ; SHEA, M. ; HAYES, W.C. ; JAISWAL, N. ; KADIYALA, S.** Bone regeneration by implantation of purified, culture-expanded human mesenchymal stem cells. *J Orthop Res,* 1998, vol. 16, 155
- **BOO, J.S. ; YAMADA, Y. ; HIBINO, Y. ; OKAZAKI, Y. ; OKADA, K. ; HATA, K. ; YOSHIKAWA, T. ; SUGIURA, Y. ; UEDA, M.** Tissue-engineered bone using mesenchymal stem cells and a biodegradable scaffold. *J Craniofac Sug,* 2002, vol. 13, 231

- **YAMADA, Y. ; BOO, J.S. ; OZAWA, R. ; NAGASAKA, T. ; OKAZAKI, Y. ; HATA, K. ; UEDA, M.** Bone regeneration following injection of mesenchymal stem cells and fibrin glue with a biodegradable scaffold. *J Cranio-Maxill Sug,* 2003, vol. 31, 27
- **ROSENBERG, M. ; MORTENSON, W.** Considerations in the corticosteroid treatment of bone cysts. *J Pediatr Orthop,* 1989, vol. 9, 240
- **SCHLAG, G. ; REDL, H.** The influence of fibrin sealant on demineralized bone matrix-dependent osteoinduction: A quantitative and qualitative study in rats. *Clin Orthop,* 1989, vol. 238, 282
- **BOWEN-POPE, D.F. ; VOGEL, A. ; ROSS, R.** Production of platelet-derived growth factor-like molecules and reduced expression of platelet-derived growth factor receptors accompany transformation by a wide spectrum of agents. *Pnas,* 1984, vol. 81, 2396
- **ROBERTS, A.B. ; SPRON, M.B.** Physiological actions and clinical applications of transforming growth factor-beta (TGF-beta. *Growth factors,* 1993, vol. 8, 1
- **ANTONAIDES, H.N. ; WILLIAMS, L.H.** Human platelet-derived growth factors: Structure and functions. *Federation Proc,* 1983, vol. 42, 2630
- **YAMADA, Y. et al.** Autogeneous Injectable Bone for Regeneration with Mesenchimal Stem Cells and Platelete-Rich Plasma:Tissu-Engineeered Bone Regeneration. *TISSUE ENGINEERING,* 2004, vol. 10 (5/6), 955-964
- **DEAN H. WHITMAN et al.** *J Oral Maxillofac Surg,* 1997, vol. 55, 1294-1299
- **JARRY J. PETERSON.** *Oral surg Oral Med Oral Pathol Oral Radiol Endod,* 1998, vol. 85, 638-646